(19) Europäisches Patentamt
European Patent Office
Office européen des brevets

(11) **EP 4 269 591 A1**

(12) **EUROPEAN PATENT APPLICATION**
published in accordance with Art. 153(4) EPC

(43) Date of publication:
**01.11.2023 Bulletin 2023/44**

(21) Application number: **20967978.6**

(22) Date of filing: **28.12.2020**

(51) International Patent Classification (IPC):
**C12N 15/53** $^{(2006.01)}$    **C12Q 1/6897** $^{(2018.01)}$

(52) Cooperative Patent Classification (CPC):
**C12N 9/0004; C12Q 1/6897**

(86) International application number:
**PCT/JP2020/049116**

(87) International publication number:
**WO 2022/144965 (07.07.2022 Gazette 2022/27)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Tohoku University
Sendai-shi, Miyagi 980-8577 (JP)**

(72) Inventors:
• **AIBA, Setsuya
Sendai-shi, Miyagi 980-8577 (JP)**
• **KIMURA, Yutaka
Sendai-shi, Miyagi 980-8577 (JP)**

(74) Representative: **Winter, Brandl - Partnerschaft
mbB
Alois-Steinecker-Straße 22
85354 Freising (DE)**

Remarks:
The complete document including Reference
Table(s) and the Sequence Listing(s) can be
downloaded from the EPO website

(54) **CHEMICAL SUBSTANCE EVALUATION SYSTEM USING MULTICOLOR LIGHT-EMITTING CELLS**

(57)    Provided is a method for evaluating immunotoxicity of a chemical substance to a mammal, including step 1: bringing a chemical substance into contact with a mammalian cell for immunotoxicity evaluation, the mammalian cell having a first reporter gene under control of a constant expression promoter and a second reporter gene under control of a promoter for immunotoxicity evaluation incorporated in a transiently or stably expressible manner; and step 2: evaluating an increase or decrease in expression of the second reporter gene relative to that of the first reporter gene and an increase or decrease in expression of the first reporter gene in the presence and absence of the chemical substance.

**Description**

Technical Field

The present invention relates to a method for evaluating immunotoxicity by directly comparing multiple transcriptional activities using mammalian cell-derived cultured cells to which multiple reporter genes are incorporated in an expressible manner. The present invention further relates to a method for screening for chemical substances, in particular, drugs that cause immunosuppression by inhibiting cell growth and/or metabolic activity.

Background Art

Humans have discovered or produced tens of millions of chemical substances, and the number of such chemical substances is thought to be increasing in a geometric progression. Some of them have harmful effects on the human body, such as allergy, autoimmunity, and immunosuppression, due to toxic effects on our immune system. Thus, screening chemical substances for immunotoxic effects is one of the essential processes for minimizing the health hazard to humans caused by chemical exposure in modern society, which is flooded with chemical substances. Currently, the gold standard for immunotoxicity evaluation is animal experiments. However, in order to comprehensively evaluate and manage tens of thousands of chemical substances, it is essential to construct an in vitro high-throughput evaluation system or an in silico evaluation system.

The present inventors developed an immunotoxicity multi-item evaluation system for chemical substances (Multi-ImmunoToxicity Assay or MITA, PTL 1). MITA includes: 1) 2H4 cells in which SLG luciferase controlled by the IL-2 promoter, SLO luciferase controlled by the IFN-γ promoter, and SLR luciferase controlled by the G3PDH promoter are incorporated in Jurkat cells; 2) THP-G1b cells in which SLG controlled by the IL-1β promoter and SLR controlled by the G3PDH promoter are incorporated in THP-1 cells; and 3) THP-G8 cells in which SLO controlled by the IL-8 promoter and SLR controlled by the G3PDH promoter are incorporated in THP-1 cells. MITA is an assay system capable of evaluating the effects of chemical substances, including drugs, on cytokine production of T cells, macrophages, or dendritic cells in a high-throughput manner.

Citation List

Patent Literature

PTL 1: WO2012/002507A

Summary of Invention

Technical Problem

Of the range of immunotoxicity, immunosuppression is also caused by inhibiting cell growth and/or metabolic activity of immunocompetent cells as well as by suppressing cytokine production. However, the method of PTL 1 is unable to evaluate chemical substances that cause immunosuppression by inhibiting cell growth and/or metabolic activity.

An object of the present invention is to provide a technique for evaluating a chemical substance that causes immunosuppression through cell growth inhibition and/or metabolic activity inhibition by using a cultured cell evaluation system in a high-throughput manner, as an alternative for conventional immunotoxicity evaluation methods using experimental animals.

Solution to Problem

In order to solve the above problem, the present inventors conducted extensive research and then revealed that in a mammalian cell for immunotoxicity evaluation having a first reporter gene under control of a constant expression promoter and a second reporter gene under control of a promoter for immunotoxicity evaluation incorporated in a transiently or stably expressible manner, the expression level of the second reporter gene relative to the expression level of the first reporter gene and the expression level of the first reporter gene serve as an indicator of chemical substances that cause immunosuppression by inhibiting cell growth and/or metabolic activity.

Additionally, the inventors also revealed that chemical substances can be evaluated with higher accuracy by combining the finding with an evaluation system using an increase or decrease in expression of the second reporter gene as an indicator.

The present invention was completed based on these findings, and includes the following broad aspects.

Item 1

A method for evaluating immunotoxicity of a chemical substance to a mammal, comprising

step 1: bringing a chemical substance into contact with a mammalian cell for immunotoxicity evaluation, the mammalian cell having a first reporter gene under control of a constant expression promoter and a second reporter gene under control of a promoter for immunotoxicity evaluation incorporated in a transiently or stably expressible manner; and

step 2: evaluating an increase or decrease in expression of the second reporter gene relative to that of the first reporter gene and an increase or decrease in expression of the first reporter gene in the presence and absence of the chemical substance.

Item 2

The method according to Item 1, wherein in step 2, when the expression level of the second reporter gene relative to the expression level of the first reporter gene in the presence of the chemical substance is increased as compared with the expression level of the second reporter gene relative to the expression level of the first reporter gene in the absence of the chemical substance, and when the expression level of the first reporter gene in the presence of the chemical substance is decreased as compared with the expression level of the first reporter gene in the absence of the chemical substance, it is determined that the chemical substance likely has cell growth inhibition action and/or metabolic activity inhibition action.

Item 3

The method according to Item 1 or 2, wherein in step 2, when the expression level of the second reporter gene relative to the expression level of the first reporter gene in the presence of the chemical substance is decreased or constant as compared with the expression level of the second reporter gene relative to the expression level of the first reporter gene in the absence of the chemical substance, and/or when the expression level of the first reporter gene in the presence of the chemical substance is increased or constant as compared with the expression level of the first reporter gene in the absence of the chemical substance, it is determined that the chemical substance unlikely has cell growth inhibition action and/or metabolic activity inhibition action.

Item 4

A method for screening an immunosuppressant comprising

step 1: bringing a chemical substance into contact with a mammalian cell for immunotoxicity evaluation, the mammalian cell having a first reporter gene under control of a constant expression promoter and a second reporter gene under control of a promoter for immunotoxicity evaluation incorporated in a transiently or stably expressible manner; and

step 2: evaluating an increase or decrease in expression of the second reporter gene relative to that of the first reporter gene and an increase or decrease in expression of the first reporter gene in the presence and absence of the chemical substance.

Item 5

The method according to Item 4, wherein in step 2, when the expression level of the second reporter gene relative to the expression level of the first reporter gene in the presence of the chemical substance is increased as compared with the expression level of the second reporter gene relative to the expression level of the first reporter gene in the absence of the chemical substance, and when the expression level of the first reporter gene in the presence of the chemical substance is decreased as compared with the expression level of the first reporter gene in the absence of the chemical substance, it is determined that the chemical substance is likely effective as an immunosuppressant.

Item 6

The method according to Item 4 or 5, wherein in step 2, when the expression level of the second reporter gene relative to the expression level of the first reporter gene in the presence of the chemical substance is decreased or constant as compared with the expression level of the second reporter gene relative to the expression level of the first reporter gene in the absence of the chemical substance, and/or when the expression level of the first reporter gene in the presence of the chemical substance is increased or constant as compared with the expression level of the first reporter gene in the absence of the chemical substance, it is determined that the chemical substance is unlikely effective as an immunosuppressant that mediates cell growth inhibition action and/or metabolic activity inhibition action.

Item 7

A method for screening an anticancer drug, comprising

step 1: bringing a chemical substance into contact with a mammalian cell for immunotoxicity evaluation, the mammalian cell having a first reporter gene under control of a constant expression promoter and a second reporter gene under control of a promoter for immunotoxicity evaluation incorporated in a transiently or stably expressible manner; and

step 2: evaluating an increase or decrease in expression of the second reporter gene relative to that of the first reporter gene and an increase or decrease in expression of the first reporter gene in the presence and absence of the chemical substance.

Item 8

The method according to Item 7, wherein in step 2, when the expression level of the second reporter gene relative to the expression level of the first reporter gene in the presence of the chemical substance is increased as compared with the expression level of the second reporter gene relative to the expression level of the first reporter gene in the absence of the chemical substance, and when the expression level of the first reporter gene in the presence of the chemical substance is decreased as compared with the expression level of the first reporter gene in the absence of the chemical substance, it is determined that the chemical substance is likely effective as an anticancer drug.

Item 9

The method according to Item 7 or 8, wherein in step 2, when the expression level of the second reporter gene relative to the expression level of the first reporter gene in the presence of the chemical substance is decreased or constant as compared with the expression level of the second reporter gene relative to the expression level of the first reporter gene in the absence of the chemical substance, and/or when the expression level of the first reporter gene in the presence of the chemical substance is increased or constant as compared with the expression level of the first reporter gene in the absence of the chemical substance, it is determined that the chemical substance is unlikely effective as an anticancer drug that mediates cell growth inhibition action and/or metabolic activity inhibition action.

Item 10

The method according to any one of Items 1 to 9, further comprising step 3: comparing and evaluating an increase or decrease in expression of the second reporter gene in the presence and absence of the chemical substance.

Item 11

The method according to any one of Items 1 to 10, wherein the mammalian cell for immunotoxicity evaluation is a Jurkat cell.

Item 12

The method according to any one of Items 1 to 11, wherein the reporter genes are a luciferase, and the maximum emission wavelength differs between the reporter genes by 20 nm or greater.

Item 13

The method according to any one of Items 1 to 12, wherein the chemical substance is at least one chemical substance selected from the group consisting of anticancer drugs, drugs that induce immunosuppression through cell growth inhibition and/or metabolic activity inhibition, immunosuppressants, and non-immunosuppressants.

Item 14

A kit for evaluating immunotoxicity of a chemical substance to a mammal, for use in the method of Item 1, 4, or 7, the kit comprising a mammalian cell for immunotoxicity evaluation, the mammalian cell having a first reporter gene under control of a constant expression promoter and a second reporter gene under control of a promoter for immunotoxicity evaluation incorporated in a transiently or stably expressible manner.

Advantageous Effects of Invention

The present invention enables the evaluation of a chemical substance that induces immunosuppression through cell growth inhibition and/or metabolic activity inhibition in a high-throughput manner. Additionally, the present invention also provides a method for screening for immunosuppressants or anticancer drugs having cell growth inhibition action and/or metabolic activity inhibition action.

Description of Embodiments

In an embodiment, the present invention provides a method for evaluating the immunotoxicity of a chemical substance to a mammal, comprising the following steps:

step 1: bringing a chemical substance into contact with a mammalian cell for immunotoxicity evaluation, the mammalian cell having a first reporter gene under control of a constant expression promoter and a second reporter gene under control of a promoter for immunotoxicity evaluation incorporated in a transiently or stably expressible manner; and

step 2: evaluating an increase or decrease in expression of the second reporter gene relative to that of the first reporter gene and an increase or decrease in expression of the first reporter gene in the presence and absence of the chemical substance.

In the present specification, examples of mammals include humans, cows, horses, sheep, monkeys, pigs, mice, rats, hamsters, guinea pigs, rabbits, and dogs, with humans being preferable.

Examples of the mammalian cells for use in the evaluation method according to an embodiment of the present invention include Jurkat cells, U937 cells, and THP-1 cells, with Jurkat cells being preferable.

The promoter for immunotoxicity evaluation, which is one of the promoters for evaluation, can be of any gene induced in response to a stimulus, and examples include cytokines, chemokines, adhesion molecules, costimulatory molecules, and histocompatibility antigens. Cytokines include promoters of genes such as IL-4, IL-2, IL-8, IL-22, IL-12p40, HO-1, IL-1β, and/or IFN-γ, with IL-2 being particularly preferred. Chemokines include promoters of genes such as CXCL1, CXCL2, CXCL3, CXCL10, CCL19, and CCL21. Adhesion molecules include promoters of genes such as CD54 and integrins. Costimulatory molecules include promoters of genes such as CD80, CD86, and PD-L1. Histocompatibility antigens include promoters of genes such as class II MHC. These promoters may be used alone, or in a combination of two or more. Preferred promoters for Jurkat cells include a combination of IL-2 and IFN-Y.

Examples of the constant expression promoter, which is another promoter for evaluation, include G3PDH, TK, and β-actin, with G3PDH being preferred. Although a single constant expression promoter is typically incorporated into a mammalian cell, two or more constant expression promoters may also be incorporated into a mammalian cell. An example of the G3PDH promoter is SEQ ID NO: 1.

An example of the promoter sequence is a sequence of 1 kb or more located upstream from the vicinity of the transcription start site optimized for immunotoxicity evaluation. More specifically, for example, SEQ ID NO: 2 (putative transcription start site: position 3,003) is the IL-2 gene promoter, and SEQ ID NO: 3 (putative transcription start site: position 4,986) is the IFN-γ gene promoter. The promoter sequences optimized for immunotoxicity evaluation desirably contain a sequence from 1 kb upstream from the transcription start site to the transcription start site. The putative transcription start site is the base at the 5' end of the Reference Sequence of the mRNA of each gene.

Examples of reporter genes include luciferases, fluorescent proteins, and colored proteins.

Examples of luciferases include luciferases derived from various luminescent organisms, such as *Vargula hilgendorfii, Acanthephyra purpurea,* luminous insects (e.g., fireflies and *Pyrearinus termitilluminans),* luminous earthworms, *Latia neritoides, Renilla reniformis,* and *Aequorea victoria* (aequorin).

Examples of fluorescent proteins include genetically modified fluorescent proteins, such as green fluorescent protein (GFP), yellow fluorescent protein (YFP), blue fluorescent protein (BFP), cyan fluorescent protein (CFP), DsRed, and red fluorescent protein (RFP).

Examples of colored proteins include phycocyanin and phycoerythrin.

Of these, luciferase genes are preferred because it is necessary to distinguish and detect signals of two or more, preferably three or more reporter genes.

In the present specification, "the expression of a reporter gene" refers to the expression of mRNA or protein of the reporter gene.

The following describes luciferase genes as reporter genes with reference to examples.

In regards to luciferase genes, because it is important to measure the amount of luminescence from two or more luciferases and calculate the relative ratio of the amounts of luminescence, two or more, preferably three or more luciferases, must produce light whose emission wavelength is substantially independent of measurement conditions (e.g., pH). If luciferases produce light that is independent of measurement conditions, and if the ratio of the amounts of luminescence of the luciferases can be measured with a color filter or like instrument, each luminescence can be quantified from the transmittance of each luminescence before and after filtering. Thus, two or more (preferably three or more) luciferases that emit light beams separable from each other are necessary.

In the present specification, the phrase "emission wavelength is substantially independent of measurement conditions" means that the variation of the maximum emission wavelength is 3 nm or less, preferably 2 nm or less, more preferably 1 nm or less, and particularly preferably 0.5 nm or less even when conditions, such as pH, temperature, or concentration, change. An amount of change in the maximum emission wavelength within these ranges is preferable because the ratio of the luciferase substances hardly changes when the expression levels of multiple luciferase substances are separated with a filter or like instrument and quantified.

When two or more luciferases produce light with the same luciferin, the condition that "emission wavelength is substantially independent of measurement conditions" is satisfied. When the luciferin is different between two or more luciferases, it is desirable that the emission wavelength is substantially independent of the measurement conditions in all of the luciferases.

In the present specification, the phrase "two or more (preferably three or more) luciferases that emit light beams separable from each other" means that the ratio of the amount of luminescence of luciferases can be measured by using, for example, a filter (e.g., a color filter, a band pass filter, a long pass filter, or a short pass filter). For example, red and green luciferases derived from *Phrixothrix hirtus,* orange and green luciferases derived from *Rhagophthalmus ohbai,* etc. can be measured for the ratio of the amount of luminescence by using a filter, except for the ratio between green color luciferases. For measuring the ratio of the amounts of luminescence, it is preferred that the maximum emission wavelengths be usually 20 nm or more, preferably 30 nm or more, more preferably 40 nm or more, and particularly preferably 50 nm or more apart from each other, depending on the performance of the filter and the peak shape of each emission spectrum. When the maximum emission wavelengths are separated to this extent, the amount of luminescence of each light emission can be quantified at the same time by measuring and converting the transmittance of each light emission before and after filtering, for example, by using a filter between the maximum wavelengths.

Preferred luciferases for use in the present invention include green to red luciferases derived from *Phrixothrix hirtus* (including the variants, maximum emission wavelength: 535 to 635 nm, for example, 540 to 630 nm), orange to green luciferases from *Pyrearinus termitilluminans* (including the variants, maximum emission wavelength: 530 to 600 nm), and orange to green luciferases from *Rhagophthalmus ohbai* (including the variants, maximum emission wavelength: 550 to 590 nm). For example, for *Phrixothrix hirtus,* a luciferase having a maximum red emission wavelength of 622 nm and a luciferase having a maximum green emission wavelength of 545 nm are known (US 2002/0119542-A1). However, the present inventors confirmed that there are a large number of luciferases that produce light between 540 to 635 nm in addition to these two types, and all of these luciferases are usable. For example, the present inventors confirmed that a red luciferase having a maximum emission wavelength of 622 nm derived from *Phrixothrix hirtus* (expressed in insects or *E. coli*) shows a shift of the maximum emission wavelength to 630 nm when expressed in mammalian cells.

In particular, when using luciferases derived from those having multiple luciferases whose maximum emission wavelengths are somewhat separated from each other, such as *Phrixothrix hirtus* or *Rhagophthalmus ohbai,* it is possible to simultaneously quantify the amounts of luminescence derived from multiple luciferases co-expressed by using a single luminescent substrate (e.g., a firefly luciferin can be used for luciferases derived from *Phrixothrix hirtus, Rhagophthalmus ohbai,* or a *Pyrearinus termitilluminans*)*,* and to thereby accurately measure the ratio of the amounts of expression of the respective promoters. For luciferases that produce light whose emission wavelength is independent of the measurement conditions (e.g., pH), the following luciferases may further be combined for use: *Renilla reniformis* luciferase that emits blue light, various kinds of luciferases of dinoflagellates (including their entire sequences or luminescent domains such as domain 1, domain 2, and domain 3; JP2002-335961A; Li L., Hong R., Hasting JW., Proc. Natl. Acad. Sci. USA (1997) 94, 8954), *Vargula hilgendorfii* luciferase, and Gaussia luciferase. For luciferases derived from *Phrixothrix hirtus, Rhagophthalmus ohbai,* or *Pyrearinus termitilluminans,* a firefly luciferin can be used, and this can decrease the background. A combination of a luciferase from dinoflagellate with a luciferin from dinoflagellate can also decrease the background. Additionally, combining a luciferase of *Vargula hilgendorfii,* which extracellularly secrets a luciferase, or Gaussia luciferase is also preferable because the activity of the cells is measured in a portion of the culture medium, and the background is thus not affected.

In a preferred embodiment of the present invention, the expression levels of at least three promoters can be quantified even with one type of luciferin by using luciferases of *Phrixothrix hirtus* and *Rhagophthalmus ohbai* (e.g., the red luciferase of *Phrixothrix hirtus* and orange and green luciferases of *Rhagophthalmus ohbai*) (VR. Viviani, A. Uchida, N. Suenaga, M. Ryufuku, and Y. Ohmiya: Thr226 is a key residue for bioluminescence spectra determination in beetle luciferases, 2001, Biochem. Biophys. Res. Communi. 280, 1286-1291). The expression levels of four or more promoters can also be quantified by adding a blue luciferase (a luciferase of *Renilla reniformis,* dinoflagellate, or *Vargula hilgendorfii*). With a good filter setting, multiple expression analyses are possible at 540 to 635 nm (green to red), preferably 540 to 630 nm. A blue luciferase for a different substrate also allows for addition of one more promoter. Thus, in simultaneous measurement using luciferases, three or more promoters can be simultaneously quantified with a single luciferin, and four or more promoters can be simultaneously quantified with different luciferins.

A preferred embodiment of the present invention is intended, for example, for increasing the number of copies of mRNA of reporter genes. Thus, examples of preferred embodiments include changing the sequence of cDNA, for example, from the codon usage of an insect (bias in the frequency of use of codons) to that for a mammal; further changing the sequence of cDNA such that no extra transcription factors bind; and further changing cDNA because the application is limited in use due to the large number of restriction enzyme sites. These techniques are also effective in expressing *Phrixothrix hirtus* luciferases or *Rhagophthalmus ohbai* luciferases in mammalian cells. In particular, it is effective to change the codon usage (bias in the frequency of use of codons) to that for a mammal, and further change the sequence of cDNA such that no extra transcription factors bind.

The sequence of cDNA can be changed taking into consideration the following points in the order of 1) to 4):

1) The amino acid sequence of luciferases should be as unchanged as possible (preferably not changed at all);
2) Next, to change the sequence of cDNA so as to prevent extra transcription factors from binding to the sequence;
3) Further, to change the codon usage of an insect in the sequence of cDNA to that for a mammal;
4) Further, to change the sequence of cDNA so as to eliminate restriction enzyme sites.

Although the above describes the expression of luciferases from *Phrixothrix hirtus* and *Rhagophthalmus ohbai,* the same also applies to luciferases derived from other organisms such as *Pyrearinus termitilluminans.*

In the present specification, the term "luciferase" includes a group of luminescent enzymes that catalyze a luciferin photochemical reaction, such as luciferases, including aequorin. Additionally, the luciferase of the present invention may also include a protein with a weak catalytic action, such as luminescent action produced by changing the structure of a luciferin (action of oxidizing a luciferin to convert it into a luminescent substance), as long as the emission wavelength is substantially independent of measurement conditions (e.g., pH).

For luciferases, a combination of two or more luciferases that produce light with the same luminescent substrate is desirable. Examples of preferred luciferases whose emission wavelength does not substantially change depending on the measurement conditions and which produce light with the same luminescent substrate include a red luciferase derived from *Phrixothrix hirtus,* a green luciferase derived from *Phrixothrix hirtus,* other luciferases having an emission wavelength within a range of about 540 to 635 nm, preferably about 540 to 630 nm derived from *Phrixothrix hirtus,* a green luciferase derived from *Rhagophthalmus ohbai,* and an orange luciferase derived from *Rhagophthalmus ohbai.* In addition to these luciferases, a luciferase derived from *Pyrearinus termitilluminans* (about 530 to 600 nm) is also included in the examples. In particular, red and green luciferases derived from *Phrixothrix hirtus* and orange and green luciferases derived from *Rhagophthalmus ohbai* exhibit an equivalent luminescence intensity as long as the amount of the luciferases is the same. This is convenient for multi-quantifying the transcriptional activity of promoters.

It is preferred that at least two luciferase genes individually produce light of different colors with the same luminescent substrate and have the similar intracellular lifespan. In this respect as well, red and green luciferases derived from *Phrixothrix hirtus* and orange and green luciferases derived from *Rhagophthalmus ohbai* are preferable, and red luciferase derived from *Phrixothrix hirtus* and orange and green luciferases derived from *Rhagophthalmus ohbai* are particularly preferable.

It is further preferred for quantification of each luminescent color by using a simple apparatus that different luminescent colors of at least one, preferably at least two luciferases whose luminescent wavelength does not change depending on the measurement conditions (e.g. pH) and of other luciferases for normalization of these luciferases used in the present invention can be separated with a filter.

In regards to a preferred combination of a promoter and a luciferase of the present invention, the constant expression promoter is monitored with the red luciferase of *Phrixothrix hirtus,* and the promoter for immunotoxicity evaluation is monitored with the orange or green luciferase of *Rhagophthalmus ohbai.* Red, orange, and green luciferases can be interchanged with each other.

The luciferase gene of the present invention for use can be a wild type or variant luciferase gene as is. DNA capable of hybridizing to a luciferase gene under stringent conditions, or DNA encoding a polypeptide of a luciferase that has one or more amino acids substituted, added, deleted, or inserted and that has luciferase activity can be used as a luciferase gene.

After studying various expression systems, the present inventors found that in order to stably express luciferases in mammalian cells, it is important to introduce an element that improves the efficiency of translation and/or an element that stabilizes mRNA into a gene construct. Accordingly, in a preferred embodiment, examples of elements that improve the efficiency of translation include a Kozak sequence (Ko), and examples of elements that stabilize mRNA include β-globin intron II. The inventors also confirmed that from the viewpoint of stable expression of luciferases in mammalian cells, it is preferable to change the sequence of cDNA from the codon usage of an insect (bias in the frequency of use of codons) to that for a mammal and to further change the sequence of cDNA such that no extra transcription factors bind.

In a preferred embodiment, the gene construct of the present invention contains a luciferase gene, a promoter upstream of the gene, an element for efficient translation, and/or an element for stabilizing mRNA, and may further contain an enhancer, IRES, SV40pA, and a drug-resistant gene (e.g., Neo$^r$).

The following are examples of preferred gene constructs of the present invention.

(1) (Promoter for immunotoxicity evaluation) - (Kozak sequence) - (Red, green, or orange luciferase) - (SV40 polyA sequence).
(2) (Promoter for immunotoxicity evaluation) - (Kozak sequence) - (Red, green, or orange luciferase) - (IRES) - (drug-resistant gene such as Neo$^r$) - (SV40 polyA sequence)

Although the gene construct of the present invention may be introduced into a mammalian cell as is, the gene construct is preferably incorporated into a vector (including, for example, a plasmid or a viral vector) and introduced into a mammalian cell. When multiple luciferases are incorporated into a gene construct in an expressible manner, a single gene construct or expression vector may be introduced into a mammalian cell. When a single luciferase is incorporated into a single gene construct, multiple gene constructs or expression vectors may be simultaneously or sequentially introduced into a mammalian cell according to an ordinary method.

In an embodiment, evaluation of immunotoxicity of a chemical substance can be performed by evaluating whether the chemical substance inhibits cell growth or metabolic activity, or both in mammalian cells for immunotoxicity evaluation that have been brought into contact with the chemical substance. Evaluation is performed by determining whether mammalian cells for immunotoxicity evaluation that have brought into contact with a chemical substance show a decrease in the following two items as compared with the mammalian cells that are not brought into contact with the chemical substance ("IL-2 Luc LTT" below).

(A) The ratio of the expression level of mRNA or protein of the second reporter gene under control of a promoter for immunotoxicity evaluation to the expression level of mRNA or protein of the first reporter gene under control of a constant expression promoter.
(B) The expression level of mRNA or protein of the first reporter gene under control of a constant expression promoter.

An increase in item (A) together with a decrease in item (B) in the above two items indicates that the chemical substance inhibits cell growth and/or metabolic activity. Thus, it is determined that the chemical substance likely has immunotoxicity. Constancy or a decrease in item (A) and/or constancy or an increase in item (B) in the above two items is determined to indicate that the chemical substance does not inhibit cell growth and/or metabolic activity. Thus, it can be determined that the chemical substance is unlikely to have immunotoxicity through cell growth inhibition and/or metabolic activity inhibition.

The chemical substance evaluated by the evaluation method in an embodiment of the present invention is not particularly limited. The chemical substance is, for example, at least one chemical substance selected from the group consisting of anticancer drugs, drugs that induce immunosuppression through cell growth inhibition and/or metabolic activity inhibition, immunosuppressants, and non-immunosuppressants.

Examples of anticancer drugs include bleomycin, etoposide, 5-fluorouracil, and Taxol. Examples of drugs that induce immunosuppression through cell growth inhibition and/or metabolic activity inhibition include azathioprine, methotrexate, mizoribine, and rapamycin. Examples of immunosuppressants include dexamethasone, cyclosporine, minocycline, and chloroquine. Examples of non-immunosuppressants include acetaminophen, indomethacin, warfarin, and pravastatin.

In a preferred embodiment, the method for evaluating a chemical substance that inhibits cell growth and/or metabolic activity is performed by using long-term stable cultured cells prepared by introducing a gene containing a polychromatic luminescent luciferase downstream of a gene promoter described below into Jurkat cells. Specifically, a red-light-emitting luciferase vector (pSLR), an orange-light-emitting luciferase vector (pSLO), and a green-light-emitting luciferase vector (pSLG), manufactured by Toyobo Co., Ltd., are preferably used. For Jurkat cells, a plasmid group containing the G3PDH gene promoter inserted into pSLR, the IL-2 gene promoter inserted into pSLG, and the IFN-γ gene promoter inserted into pSLO is used. A stable cell line, Jurkat 2H4 cells, having the plasmid group introduced, can be prepared. The promoter sequences of the IL-2 gene and the IFN-γ gene contain 1 kb, more preferably 3 kb or more, upstream of the transcription start site. More specifically, in regards to the IL-2 gene promoter, the sequence of 3 kb upstream is described in SEQ ID NO: 2, and the transcription start site is estimated to be around position 3,003. Thus, "1 kb upstream" means a sequence downstream of around position 2,003, and "3 kb upstream" means a sequence downstream of around position 3. In regards to the IFN-γ gene promoter, the sequence of 5 kb upstream is described in SEQ ID NO: 3, and the transcription start site is estimated to be around position 4,986. Thus, the sequence of "1 kb upstream" means a sequence downstream of around position 3,986, and the sequence of "3 kb upstream" means a sequence downstream of around position 1,986.

In a preferred embodiment, the evaluation of a chemical substance is performed as follows. Mammalian cells for immunotoxicity evaluation are treated or brought into contact with a chemical substance in a concentration of, for example, 0.1 ng/mL or more and 2 mg/mL or less. After 18 to 30 hours, the mammalian cells are stimulated with PMA (Phorbol 12-myristate 13-acetate) and ionomycin. After the mammalian cells are cultured for 5 to 10 hours, the activities of three luciferases, namely an orange-light-emitting luciferase (SLO), a green-light-emitting luciferase (ISLR), and an orange-light-emitting luciferase (SLG), are quantified, and the results are compared with those of the mammalian cells that have no contact with the chemical substance in regards to the above items (A) and (B). More preferably, determination is made based on the following indicator. In (A-1) and (B-1), "nIL2LA" is a value determined by normalizing SLG luciferase activity (IL2LA) with SLR luciferase activity (GAPLA). (A-1)% suppression = {(nIL2LA in 2H4 cells untreated with a chemical substance) - (nIL2LA in 2H4 cells treated with a chemical substance)} $\times$ 100/(nIL2LA in 2H4 cells untreated with a chemical substance).

(B-1) Inh-GAPLA = (GAPLA in 2H4 cells treated with a chemical substance)/(GAPLA in 2H4 cells untreated with a chemical substance).

If the minimum values of (A-1) and (B-1) are both equal to or lower than the reference value, it is determined that the chemical substance inhibits cell growth and/or metabolic activity. If at least one of the above two items is equal to or greater than the reference value, it is determined that the chemical substance does not inhibit either cell growth or metabolic activity.

(A-1) The reference value of % suppression is preferably set in a range of -50% to -20%, more preferably in a range of -40% to -30%, and particularly preferably -35%. (B-1) The reference value of Inh-GAPLA is preferably set in a range of 0.5 to 0.9, more preferably in a range of 0.6 to 0.8, and particularly preferably 0.7.

In the above evaluation method, a chemical substance that has a value of (A-1) % suppression in the range of -35% to 35% and a value of (B-1) Inh-GAPLA in the range of 0.7 to 1.0 may be insoluble in water, and thus can be determined as "unevaluable."

The reliability of the evaluation method can be confirmed by using SLO luciferase activity (IFNLA) in 2H4 cells untreated with a chemical substance.

In a preferred embodiment, a chemical substance can be evaluated with higher accuracy by further performing the following evaluation (C) in addition to evaluations (A) and (B) (preferably (A-1) and (B-1)). Evaluation is performed based on whether mammalian cells for immunotoxicity evaluation that have been brought into contact with a chemical substance show an increase in the following item (C) as compared with the mammalian cell that have not brought into contact with the chemical substance. Evaluation (C) may be referred to as "IL-2 Luc assay."

(C) The step of comparing and evaluating an increase or decrease in expression of the second reporter gene in the presence and absence of the chemical substance.

An increase (in particular, a significant increase) in item (C) can be determined to indicate that the chemical substance is likely to have immunotoxicity. A decrease or constancy in item (C) can be determined to indicate that the chemical substance is unlikely to have immunotoxicity.

In evaluation using the 2H4 cells described above, evaluation is performed as follows. For example, 2H4 cells are treated with a chemical substance in a concentration of 0.1 ng/mL or more and 2 mg/mL or less and then stimulated with PMA and ionomycin. After the cells are cultured for 6 to 12 hours, the luciferase activities of the three colors SLO, SLR, and SLG are quantified and compared with those in 2H4 cells that are not brought into contact with the chemical substance. More preferably, evaluation can be performed by using the value of (A-1) % suppression. If the value is equal to or greater than a reference value set separately from the value described above, it is determined that the chemical substance is immunosuppressive. The reference value in this case is preferably set in the range of 20% to 50%, more preferably in the range of 30% to 40%, and particularly preferably 35%.

In the above method, if the value is equal to or lower than a reference value set separately, it can be determined that the chemical substance has an immunoaugmenting effect. The reference value for this evaluation is preferably set in the range of -20% to -50%, more preferably in the range of -30% to -40%, and particularly preferably -35%.

In another embodiment of the present invention, a method for screening an immunosuppressant is provided. The method for screening an immunosuppressant includes

step 1: bringing a chemical substance into contact with a mammalian cell for immunotoxicity evaluation, the mammalian cell having a first reporter gene under control of a constant expression promoter and a second reporter gene under control of a promoter for immunotoxicity evaluation incorporated in a transiently or stably expressible manner; and

step 2: evaluating an increase or decrease in expression of the second reporter gene relative to that of the first reporter gene and an increase or decrease in expression of the first reporter gene in the presence and absence of the chemical substance.

The mammalian cell for immunotoxicity evaluation, the two or more reporter genes, the constant expression promoter, and the promoter for immunotoxicity evaluation are as described above for the method for evaluating immunotoxicity of a chemical substance to a mammal.

In step 2, when the expression level of the second reporter gene relative to the expression level of the first reporter gene in the presence of the chemical substance is increased as compared with the expression level of the second reporter gene relative to the expression level of the first reporter gene in the absence of the chemical substance, and when the expression level of the first reporter gene in the presence of the chemical substance is decreased as compared with the expression level of the first reporter gene in the absence of the chemical substance, it is indicated that the chemical substance inhibits cell growth and/or metabolic activity. Thus, it is determined that the chemical substance is likely an effective immunosuppressant.

In step 2, when the expression level of the second reporter gene relative to the expression level of the first reporter gene in the presence of the chemical substance is decreased or constant as compared with the expression level of the second reporter gene relative to the expression level of the first reporter gene in the absence of the chemical substance, and/or when the expression level of the first reporter gene in the presence of the chemical substance is increased or constant as compared with the expression level of the first reporter gene in the absence of the chemical substance, it is determined that the chemical substance is unlikely effective as an immunosuppressant that mediates cell growth inhibition action and/or metabolic activity inhibition action.

In another embodiment of the present invention, a method for screening an anticancer drug is provided. The method for screening an anticancer drug includes

step 1: bringing a chemical substance into contact with a mammalian cell for immunotoxicity evaluation, the mammalian cell having a first reporter gene under control of a constant expression promoter and a second reporter gene under control of a promoter for immunotoxicity evaluation incorporated in a transiently or stably expressible manner; and

step 2: evaluating an increase or decrease in expression of the second reporter gene relative to that of the first reporter gene and an increase or decrease in expression of the first reporter gene in the presence and absence of the chemical substance.

The mammalian cell for immunotoxicity evaluation, the two or more reporter genes, the constant expression promoter, and the promoter for immunotoxicity evaluation are as described above for the method for evaluating immunotoxicity of a chemical substance to a mammal.

In step 2, when the expression level of the second reporter gene relative to the expression level of the first reporter gene in the presence of the chemical substance is increased as compared with the expression level of the second reporter gene relative to the expression level of the first reporter gene in the absence of the chemical substance, and when the expression level of the first reporter gene in the presence of the chemical substance is decreased as compared with the expression level of the first reporter gene in the absence of the chemical substance, it is indicated that the chemical substance inhibits cell growth and/or metabolic activity. Thus, it is determined that the chemical substance is likely an effective anticancer drug.

In step 2, when the expression level of the second reporter gene relative to the expression level of the first reporter gene in the presence of the chemical substance is decreased or constant as compared with the expression level of the second reporter gene relative to the expression level of the first reporter gene in the absence of the chemical substance, and/or when the expression level of the first reporter gene in the presence of the chemical substance is increased or constant as compared with the expression level of the first reporter gene in the absence of the chemical substance, it is determined that the chemical substance is unlikely an effective anticancer drug that mediates cell growth inhibition action and/or metabolic activity inhibition action.

In another embodiment of the present invention, a kit for evaluating immunotoxicity of a chemical substance to a mammal is provided. The kit of the present invention contains a mammalian cell for immunotoxicity evaluation, and the mammalian cell has a first reporter gene under control of a constant expression promoter and a second reporter gene under control of a promoter for immunotoxicity evaluation incorporated in a transiently or stably expressible manner. The kit may further contain PMA (phorbol 12-myristate 13-acetate) and ionomycin. The kit preferably contains PMA and ionomycin because of their effect of activating cells. Additionally, the kit may also contain other reagents necessary for immunotoxicity evaluation, a welled plate, and instruments, such as tubes. Such a kit can be used in the method for evaluating immunotoxicity of a chemical substance to a mammal in the embodiments of the present invention.

Examples

The following describes the present invention in more detail with reference to Examples. However, the present invention is not limited to the Examples.

List of Chemical Substances Used in Study

Table 1 lists the chemical substances studied in the Examples.

Table 1: CAS Number of Studied Chemical Substances, Concentration Examined with Suppliers, and Solvents Used

| No | Chemicals | Cas No. | Supplier | Concentration (mg/mL) | Vehicle |
|---|---|---|---|---|---|
| 1 | 5-Fluorouracil | 51-21-8 | WAKO | 0.1-63 | DMSO |
| 2 | 6-Mercaptoprine | 50-44-2 | Sigma | 0.05-25 | DMSO |
| 3 | 6-Thioguanine | 154-42-7 | TCI | 0.1-63 | DMSO |
| 4 | Acetaminophen (AA) | 103-90-2 | Sigma | 1-500 | DMSO |
| 5 | Acyclovir | 59277-89-3 | TCI | 0.12-62 | DMSO |
| 6 | Azathioprine (AZ) | 446-86-6 | Sigma | 0.16-80 | DMSO |
| 7 | Bleomycin sulfate | 9041-93-4 | TCI | 0.4-200 | ddw |
| 8 | Busulfan | 55-98-1 | Supelco | 0.3-125 | DMSO |
| 9 | Carmustine | 154-93-8 | TCI | 0.2-100 | DMSO |
| 10 | Chloroquine (CQ) | 50-63-5 | Sigma | 2-1000 | ddw |
| 11 | Chlorothirazide | 58-94-6 | TCI | 0.4-200 | DMSO |
| 12 | Colchicine | 64-86-8 | Sigma | 0.4-2000 | ddw |
| 13 | Cyclosporin A (CyA) | 59865-13-3 | Sigma | 0.002-1 | DMSO |
| 14 | Cytarabine | 147-94-4 | TCI | 0.02-2000 | ddw |
| 15 | Dexamethasone (Dex) | 50-02-2 | WAKO | 1-500 | DMSO |
| 16 | Digoxin | 20830-75-5 | Supelco | 0.0004-50 | DMSO |
| 17 | Diphenhydramine | 147-24-4 | Sigma | 0.4-200 | ddw |
| 18 | Doxorubicin hydrochloride | 25316-40-9 | TCI | 0.05-25 | DMSO |
| 19 | Etoposide | 33419-42-0 | TCI | 0.2-100 | DMSO |
| 20 | Fludarabine | 21679-14-1 | TCI | 0.5-250 | DMSO |
| 21 | Gemcitabine hydrochloride | 122111-03-9 | TCI | 0.008-1000 | ddw |
| 22 | Indomethacin | 53-86-1 | Sigma | 0.2-500 | DMSO |
| 23 | Igratimod | 123663-49-0 | TCI | 0.05-25 | DMSO |
| 24 | Isoniazid | 54-85-3 | Supelco | 4-2000 | ddw |
| 25 | Lansoprazole | 103577-45-3 | TCI | 0.2-200 | DMSO |
| 26 | Leflunomide | 75706-12-6 | Sigma | 0.05-25 | DMSO |
| 27 | Lithium carbonate | 554-13-2 | WAKO | 0.4-200 | ddw |
| 28 | Mannitol | 69-65-8 | Sigma | 4-2000 | ddw |
| 29 | Methotrexate (MTX) | 13307-73-1 | Sigma | 0.09-200 | DMSO |
| 30 | Minocycline (MC) | 13614-98-7 | Sigma | 1-500 | ddw |

(continued)

| No | Chemicals | Cas No. | Supplier | Concentration (mg/mL) | Vehicle |
|---|---|---|---|---|---|
| 31 | Mizoribine (MZR) | 50924-49-7 | Sigma | 0.1-52 | ddw |
| 32 | Mycophenolic acid (MPA) | 24280-93-1 | Sigma | 0.2-96 | DMSO |
| 33 | Nicotinamide (NA) | 98-92-0 | Sigma | 20-10000 | ddw |
| 34 | Paclitaxel | 33069-62-4 | WAKO | 0.0002-25 | DMSO |
| 35 | Pirfenidone | 53179-13-8 | TCI | 0.05-25 | DMSO |
| 36 | Pravastatin | 81131-70-6 | TCI | 0.4-200 | ddw |
| 37 | Rapamycin (RPM) | 53123-88-9 | Sigma | 0.002-0.9 | DMSO |
| 38 | Retrovir | 30516-87-1 | TCI | 0.8-400 | ddw |
| 39 | Sulfasalazine (SASP) | 599-79-1 | Sigma | 0.24-120 | DMSO |
| 40 | Tacrolimus (Tac) | 109581-93-3 | Sigma | 0.003-1.6 | DMSO |
| 41 | Teniposide | 29767-20-2 | TCI | 0.2-100 | DMSO |
| 42 | Terbinafine | 78628-80-5 | TCI | 0.1-50 | DMSO |
| 43 | Warfarin | 81-81-2 | Supelco | 1-500 | DMSO |

Example 1: Establishment of 2H4 Cells

2H4 cells were established from Jurkat cells according to the method described in PTL 1. First, for the preparation of 2H4 cells, a plasmid having SLG introduced downstream of the IL-2 promoter, a plasmid having SLR introduced downstream of the G3PDH promoter, and a plasmid having SLO introduced downstream of the INF-γ promoter were prepared as internal standards. These plasmids contained a resistance gene for hygromycin B, puromycin, and neomycin downstream of SLG, SLR, and SLO, respectively. The conditions for drug selection after transfection of the host Jurkat cells were as follows: in the primary selection, the cells were cultured for 1 week in the presence of a drug. In the secondary selection culture, the cells that survived in the primary selection culture were seeded in a 96-well plate at 1 cell/well or 200 cells/well, and cultured for 2 weeks under the same drug conditions. After the secondary selection culture, the cells were subjected to clone selection from the two points of signal intensity and signal induction by drug treatment. The G3PDH promoter SLR was first introduced to establish monochromatic stable cell line Jurkat: G3PDH-SLR #14D1 cells. These cells were used as the parent cell line to introduce the INF-γ promoter SLO, thereby establishing bichromatic stable cell line Jurkat: INFγ-SLO/G3PDH-SLR #2B12 cells. Subsequently, the obtained cells were used as a parent cell line to introduce the IL-2 promoter SLG, thereby establishing trichromatic stable cell line Jurkat IL-2-SLG/INFγ-SLO/G3PDH-SLR #2H4 cells.

Example 2: Evaluation of Chemical Substance in 2H4 Cell (IL-2 Luc LTT)

The chemical substances listed in Table 1 were evaluated using 2H4 cells. 2H4 cells were seeded in a 96-well plate at $1 \times 10^4$/well and treated with each chemical substance for 1 hour, followed by incubating the cells with 25 nM PMA and 1 μM ionomycin for 24 hours. After 24 hours from the treatment with PMA and ionomycin, Tripluc® Assay Reagent (manufactured by Toyobo Co., Ltd.) was added, and the amount of luminescence was measured with a Phelios microplate luminometer (manufactured by Atto Corporation). For control, 2H4 cells untreated with a chemical substance were also examined in the same manner. The amount of luminescence of SLG luciferase activity (IL2LA), SLR luciferase activity (GAPLA), and SLO luciferase activity (IFNLA) was measured, and IL-2LA and IFNLA were each normalized by GAPLA and determined to be nIL2LA and nIFNLA, respectively. Thereafter, for the concentration of each chemical substance, "% suppression" and "Inh-GAPLA" were determined according to the following formulas (1) and (2), and the chemical substances were evaluated by using their minimum value (Min % suppression, Min Inh-GAPLA).

In the evaluation, for control, PMA/ionomycin-treated 2H4 cells and PMA/ionomycin-untreated 2H4 cells were also measured for nIL2LA. When the ratio of nIL2LA in PMA/ionomycin-treated 2H4 cells to nIL2LA in PMA/ionomycin-untreated 2H4 cells was lower than 3.0, the result was not used.

```
% suppression = {(nIL2LA in 2H4 cells untreated with a chemical
substance) - (nIL2LA in 2H4 cells treated with a chemical
substance)} × 100/(nIL2LA in 2H4 cells untreated with a chemical
substance) --- (1)

    Inh-GAPLA = (GAPLA in 2H4 cells treated with a chemical
substance)/(GAPLA in 2H4 cells untreated with a chemical
substance)  --- (2)
```

Example 3: Evaluation of Chemical Substance in 2H4 Cell (IL-2 Luc Assay)

The chemical substances listed in Table 1 were evaluated using 2H4 cells. 2H4 cells were seeded in a 96-well plate at $2 \times 10^5$ cells/50 pl/well and treated with each chemical substance for 1 hour, followed by incubating the cells with 25 nM PMA and 1 pM ionomycin for 24 hours. After six hours from the treatment with PMA and ionomycin, Tripluc® Assay Reagent (manufactured by Toyobo Co., Ltd.) was added, and the amount of luminescence was measured with a Phelios microplate luminometer (manufactured by Atto Corporation). For control, 2H4 cells untreated with a chemical substance were also examined in the same manner. The amount of luminescence of SLG luciferase activity (IL2LA), SLR luciferase activity (GAPLA), and SLO luciferase activity (IFNLA) was measured, and IL-2LA and IFNLA were each normalized by GAPLA and determined to be nIL2LA and nIFNLA, respectively. Thereafter, for the concentration of each chemical substance, the value of % suppression was calculated according to formula (1) above, and the chemical substances were evaluated by using their minimum value (Min % suppression).

Evaluation Method

IL-2 Luc LTT

Min % suppression ≤ -35 and Min Inh-GAPLA < 0.7: Leukocyte toxic (denoted as "LCT"; determined to have cell growth inhibition action and/or metabolic activity inhibition action)

-35 < Min % suppression < 35 and 0.7 ≤ Min Inh-GAPLA:
Inconclusive (denoted as "I"; possibly not measurable due to low water solubility)
Other than the above: Non-leukocyte toxic (denoted as "NLCT";
determined to have no cell growth inhibition action and metabolic activity inhibition action)

IL-2 Luc assay

Min % suppression ≥ 35: Suppression (denoted as "S"; determined to have immunosuppressive action)
Min % suppression ≤ -35: Augmentation (denoted as "A"; determined to have immunoaugmenting action)
-35 < Min % suppression < 35: No Effect (denoted as "N"; determined to not affect immunity)

Evaluation of Combination

Positive (Pos): If the result is either LCT in the IL-2 Luc LTT or S in the IL-2 Luc assay, or both, the chemical substance is determined to be an immunosuppressive substance.
Negative (Neg): In the case other than the above, the chemical substance is determined to be a non-immunosuppressive substance.

Evaluation Results

Table 2 shows the results. Mizoribine, mycophenolic acid, and rapamycin, which could not be evaluated by the IL-2 Luc assay alone (i.e., these were determined to not have immunosuppressive action), were accurately determined to be chemical substances that inhibit cell growth and/or metabolic activity in IL-2 Luc LTT. In the evaluation results of chemical substances Nos. 1 to 19 (anticancer drugs and immunosuppressants that inhibit cell growth and/or metabolic activity), 12 chemical substances were correctly evaluated as LCT in the IL-2 Luc LTT; the results were more accurate than the results in the IL-2 Luc assay (5 chemical substances). Additionally, the combination of IL-2 Luc LTT and IL-2 Luc assay was able to determine that 24 chemical substances out of 28 chemical substances (Nos. 1 to 28) were immunotoxic. Given the results of 14 chemical substances in the IL-2 Luc LTT alone and the results of 15 chemical substances in the IL-2 Luc assay alone, the combination of IL-2 Luc LTT and IL-2 Luc assay appears to be able to perform evaluation with higher accuracy.

```
Table 2: Determination Results of Chemical Substances in IL-2 Luc
Assay and IL-2 Luc LTT
```

| No. | Classification | Chemicals | IL-2 Luc LTT | IL-2 Luc Assay | Evaluation of Combination |
|---|---|---|---|---|---|
| 1 | Anticancer Drug | Doxorubicin hydrochloride | LCT | S | Pos |
| 2 | | Bleomycin sulfate | LCT | N | Pos |
| 3 | | Busulfan | I | N | Neg |
| 4 | | Carmustine | NLCT | S | Pos |
| 5 | | Cytarabine | LCT | N | Pos |
| 6 | | Etoposide | LCT | N | Pos |
| 7 | | Fludarabine | LCT | A | Pos |
| 8 | | 5-Fluorouracil | LCT | N | Pos |
| 9 | | Gemcitabine hydrochloride | NLCT | N | Pos |
| 10 | | Paclitaxel | LCT | S | Pos |
| 11 | | Teniposide | LCT | N | Pos |
| 12 | | 6-Thioguanine | LCT | N | Pos |
| 13 | Immunosuppressants that inhibit cell growth and metabolic activity | Azathioprine (AZ) | NLCT | S | Pos |
| 14 | | 6-Mercaptoprine | NLCT | N | Neg |
| 15 | | Leflunomide | NLCT | S | Pos |
| 16 | | Methotrexate (MTX) | NLCT | N | Neg |
| 17 | | Mizoribine (MZR) | LCT | N | Pos |
| 18 | | Mycophenolic acid (MPA) | LCT | A | Pos |
| 19 | | Rapamycin (RPM) | LCT | N | Pos |
| 20 | Immunosuppressants other than those above | Dexamethasone (Dex) | NLCT | N | Neg |
| 21 | | Cyclosporin A (CyA) | NLCT | S | Pos |
| 22 | | Tacrolimus (Tac) | NLCT | S | Pos |
| 23 | | Igratimod | NLCT | S | Pos |
| 24 | | Colchicine | LCT | S | Pos |
| 25 | | Sulfasalazine (SASP) | NLCT | S | Pos |
| 26 | | Minocycline (MC) | NLCT | S | Pos |
| 27 | | Chloroquine (CQ) | NLCT | S | Pos |
| 28 | | Nicotinamide (NA) | LCT | A | Pos |
| 29 | Chemical substances with no immunosuppression | Acetaminophen (AA) | LCT | A | Pos |
| 30 | | Indomethacin | LCT | S | Pos |
| 31 | | Pirfenidone | I | S | Pos |
| 32 | | Digoxin | NLCT | S | Pos |
| 33 | | Warfarin | NLCT | N | Neg |
| 34 | | Lithium carbonate | I | N | Neg |
| 35 | | Acyclovir | I | N | Neg |
| 36 | | Retrovir | NLCT | A | Pos |
| 37 | | Isoniazid | I | N | Neg |
| 38 | | Terbinafine | NLCT | N | Pos |
| 39 | | Diphenhydramine | NLCT | S | Pos |
| 40 | | Chlorothiazide | NLCT | N | Neg |
| 41 | | Pravastatin | I | N | Neg |
| 42 | | Lansoprazole | NLCT | S | Pos |
| 43 | | Mannitol | I | N | Neg |

LCT: Leukocyte Toxic

NLCT: Non-leukocyte Toxic

```
I: Inconclusive
S: Suppression
A: Augmentation
N: No Effect
Pos: Immunosuppressive Substance
Neg: Non-immunosuppressive Substance
```

**Claims**

1. A method for evaluating immunotoxicity of a chemical substance to a mammal, comprising

   step 1: bringing a chemical substance into contact with a mammalian cell for immunotoxicity evaluation, the mammalian cell having a first reporter gene under control of a constant expression promoter and a second reporter gene under control of a promoter for immunotoxicity evaluation incorporated in a transiently or stably expressible manner; and
   step 2: evaluating an increase or decrease in expression of the second reporter gene relative to that of the first reporter gene and an increase or decrease in expression of the first reporter gene in the presence and absence of the chemical substance.

2. The method according to claim 1, wherein in step 2, when the expression level of the second reporter gene relative to the expression level of the first reporter gene in the presence of the chemical substance is increased as compared with the expression level of the second reporter gene relative to the expression level of the first reporter gene in the absence of the chemical substance, and when the expression level of the first reporter gene in the presence of the chemical substance is decreased as compared with the expression level of the first reporter gene in the absence of the chemical substance, it is determined that the chemical substance likely has cell growth inhibition action and/or metabolic activity inhibition action.

3. The method according to claim 1 or 2, wherein in step 2, when the expression level of the second reporter gene relative to the expression level of the first reporter gene in the presence of the chemical substance is decreased or constant as compared with the expression level of the second reporter gene relative to the expression level of the first reporter gene in the absence of the chemical substance, and/or when the expression level of the first reporter gene in the presence of the chemical substance is increased or constant as compared with the expression level of the first reporter gene in the absence of the chemical substance, it is determined that the chemical substance unlikely has cell growth inhibition action and/or metabolic activity inhibition action.

4. A method for screening an immunosuppressant comprising

   step 1: bringing a chemical substance into contact with a mammalian cell for immunotoxicity evaluation, the mammalian cell having a first reporter gene under control of a constant expression promoter and a second reporter gene under control of a promoter for immunotoxicity evaluation incorporated in a transiently or stably expressible manner; and
   step 2: evaluating an increase or decrease in expression of the second reporter gene relative to that of the first reporter gene and an increase or decrease in expression of the first reporter gene in the presence and absence of the chemical substance.

5. The method according to claim 4, wherein in step 2, when the expression level of the second reporter gene relative to the expression level of the first reporter gene in the presence of the chemical substance is increased as compared with the expression level of the second reporter gene relative to the expression level of the first reporter gene in the absence of the chemical substance, and when the expression level of the first reporter gene in the presence of the chemical substance is decreased as compared with the expression level of the first reporter gene in the absence of the chemical substance, it is determined that the chemical substance is likely effective as an immunosuppressant.

6. The method according to claim 4 or 5, wherein in step 2, when the expression level of the second reporter gene relative to the expression level of the first reporter gene in the presence of the chemical substance is decreased or constant as compared with the expression level of the second reporter gene relative to the expression level of the first reporter gene in the absence of the chemical substance, and/or when the expression level of the first reporter gene in the presence of the chemical substance is increased or constant as compared with the expression level of the first reporter gene in the absence of the chemical substance, it is determined that the chemical substance is unlikely effective as an immunosuppressant that mediates cell growth inhibition action and/or metabolic activity inhibition action.

7. A method for screening an anticancer drug, comprising

step 1: bringing a chemical substance into contact with a mammalian cell for immunotoxicity evaluation, the mammalian cell having a first reporter gene under control of a constant expression promoter and a second reporter gene under control of a promoter for immunotoxicity evaluation incorporated in a transiently or stably expressible manner; and

step 2: evaluating an increase or decrease in expression of the second reporter gene relative to that of the first reporter gene and an increase or decrease in expression of the first reporter gene in the presence and absence of the chemical substance.

8. The method according to claim 7, wherein in step 2, when the expression level of the second reporter gene relative to the expression level of the first reporter gene in the presence of the chemical substance is increased as compared with the expression level of the second reporter gene relative to the expression level of the first reporter gene in the absence of the chemical substance, and when the expression level of the first reporter gene in the presence of the chemical substance is decreased as compared with the expression level of the first reporter gene in the absence of the chemical substance, it is determined that the chemical substance is likely effective as an anticancer drug.

9. The method according to claim 7 or 8, wherein in step 2, when the expression level of the second reporter gene relative to the expression level of the first reporter gene in the presence of the chemical substance is decreased or constant as compared with the expression level of the second reporter gene relative to the expression level of the first reporter gene in the absence of the chemical substance, and/or when the expression level of the first reporter gene in the presence of the chemical substance is increased or constant as compared with the expression level of the first reporter gene in the absence of the chemical substance, it is determined that the chemical substance is unlikely effective as an anticancer drug that mediates cell growth inhibition action and/or metabolic activity inhibition action.

10. The method according to any one of claims 1 to 9, further comprising step 3: comparing and evaluating an increase or decrease in expression of the second reporter gene in the presence and absence of the chemical substance.

11. The method according to any one of claims 1 to 10, wherein the mammalian cell for immunotoxicity evaluation is a Jurkat cell.

12. The method according to any one of claims 1 to 11, wherein the reporter genes are a luciferase, and the maximum emission wavelength differs between the reporter genes by 20 nm or greater.

13. The method according to any one of claims 1 to 12, wherein the chemical substance is at least one chemical substance selected from the group consisting of anticancer drugs, drugs that induce immunosuppression through cell growth inhibition and/or metabolic activity inhibition, immunosuppressants, and non-immunosuppressants.

14. A kit for evaluating immunotoxicity of a chemical substance to a mammal, for use in the method of claim 1, 4, or 7, the kit comprising a mammalian cell for immunotoxicity evaluation, the mammalian cell having a first reporter gene under control of a constant expression promoter and a second reporter gene under control of a promoter for immunotoxicity evaluation incorporated in a transiently or stably expressible manner.

**EP 4 269 591 A1**

## INTERNATIONAL SEARCH REPORT

| | International application No. |
|---|---|
| | PCT/JP2020/049116 |

**A. CLASSIFICATION OF SUBJECT MATTER**
C12N 15/53(2006.01)i; C12Q 1/6897(2018.01)i
FI: C12N15/53; C12Q1/6897 Z
According to International Patent Classification (IPC) or to both national classification and IPC

**B. FIELDS SEARCHED**

Minimum documentation searched (classification system followed by classification symbols)
C12N15/53; C12Q1/6897

Documentation searched other than minimum documentation to the extent that such documents are included in the fields searched
| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | KIMURA, Y. et al., "An international validation study of the IL-2 Luc assay for evaluating the potential immunotoxic effects of chemicals on T cells and a proposal for reference data for immunotoxic chemicals", Toxicology in Vitro, (2020), vol. 66, no. 104832, pp. 1-8, published online 18 March 2020, abstract, Materials and methods, table 1 | 1-14<br>1-13 |
| X<br>Y | WO 2012/002507 A1 (TOHOKU UNIVERSITY et al.) 05 January 2012 (2012-01-05) claims, examples | 1-14<br>1-13 |
| Y | KIMURA, Y. et al., "The modified IL-8 Luc assay, an in vitro skin sensitisation test, can significantly improve the false-negative judgment of lipophilic sensitizers with logKow values>3.5", Archives of Toxicology, (2021), vol. 95, pp. 749-758, published online 17 October 2020, abstract, Materials and methods, tables 1, 2 | 1-13 |

☐ Further documents are listed in the continuation of Box C.  ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 24 February 2021 (24.02.2021) | 09 March 2021 (09.03.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**
Information on patent family members

International application no.

PCT/JP2020/049116

| Patent Documents referred in the Report | Publication Date | Patent Family | Publication Date |
|---|---|---|---|
| WO 2012/002507 A1 | 05 Jan. 2012 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012002507 A
- US 20020119542 A1

- JP 2002335961 A

**Non-patent literature cited in the description**

- **LI L. ; HONG R. ; HASTING JW.** *Proc. Natl. Acad. Sci. USA,* 1997, vol. 94, 8954

- **VR. VIVIANI ; A. UCHIDA ; N. SUENAGA ; M. RYUFUKU ; Y. OHMIYA.** Thr226 is a key residue for bioluminescence spectra determination in beetle luciferases. *Biochem. Biophys. Res. Communi.,* 2001, vol. 280, 1286-1291